# FASCICULE DE BREVET EUROPEEN

(11) **EP 0 706 354 B1**
(45) Date de publication et mention de la délivrance du brevet: **17.02.1999**
(21) Numéro de dépôt: 94920517.3
(22) Date de dépôt: 27.06.1994
(51) Int. Cl.: A61F 2/44

(54) **PROTHESE DISCALE INTERVERTEBRALE**
INTERVERTEBRALE SCHEIBENPROTHESE
INTERVERTEBRAL DISK PROSTHESIS

(30) Priorité: 28.06.1993 FR 9307855
(43) Date de publication de la demande: 17.04.1996
(73) Titulaire: BISSERIE, Michel, F-75009Paris (FR)
(72) Inventeur: BISSERIE, Michel, F-75009Paris (FR)
(74) Mandataire: Michelet, Alain
(86) Numéro de dépôt international: FR9400774
(87) Numéro de publication internationale: WO9500082

(56) Documents cités:
- EP-A- 0 392 076
- WO-A-90/11740
- FR-A- 1 122 634
- US-A- 4 772 287

## Description

La présente invention concerne une prothèse interne à application humaine, destinée à assurer le remplacement d'un disque intervertébral déficient au niveau dorsal bas, et surtout lombaire et lombo-sacré.

Le souci général de base de la chirurgie ostéo-articulaire est de réparer des lésions et, éventuellement, de prévenir leur aggravation, dans le but de préserver autant que faire se peut les deux fonctions des structures ostéo-articulaires, fonction de soutien d'une part, et fonction de mouvement d'autre part.

Les motivations d'un acte chirurgical au niveau de la colonne vertébrale actuellement résident, dans la majorité des cas, dans la détérioration mécanique des structures de soutien intervertébrales, dont la principale est le disque intervertébral.

L'évolution de cette détérioration entraîne le plus souvent deux conséquences néfastes :
- d'une part, des douleurs et une limitation des facultés de mouvement au niveau de la colonne vertébrale elle-même ;
- d'autre part, une atteinte par compression mécanique des structures neurologiques contenues dans le canal vertébral, ce qui entraîne des déficiences périphériques.

La chirurgie vertébrale doit donc, dans ce cas, s'efforcer d'atteindre un triple but :
1) restituer la fonction de soutien des structures intervertébrales ;
2) restituer la fonction de mouvement de ces mêmes structures ;
3) libérer les structures neurologiques dont l'intégrité est compromise.

Il se trouve que la configuration anatomique particulière de la colonne vertébrale n'autorise pas les techniques chirurgicales actuelles à atteindre ce triple but par un seul protocole opératoire.

En effet :
- d'une part, on ne peut généralement traiter correctement les lésions neurologiques que par un abord chirurgical postérieur, auquel cas on ne peut qu'effectuer des actes palliatifs sur le disque intervertébral qui restera déficient, ou bien le supprimer en fusionnant les vertèbres par des greffes osseuses, ce qui assure le soutien, mais supprime définitivement le mouvement ;
- d'autre part et jusqu'à présent, on ne peut remplacer le disque intervertébral par une prothèse mobile que par un abord chirurgical antérieur qui, généralement, ne permet pas d'assurer correctement, dans le même temps, le traitement des lésions neurologiques postérieures susmentionnées.

En effet, les prothèses discales connues actuellement assurent un remplacement discal total monobloc qui ne peut être techniquement réalisé que par un abord antérieur.

Par ailleurs, les prothèses discales actuelles sont limitatives en ce qui concerne les mouvements, et ne présentent pas toutes les propriétés mécaniques permettant d'assurer l'ensemble des mobilités Souhaitables dans les trois plans de l'espace et leurs combinaisons, nécessaires au fonctionnement physiologique d'un étage intervertébral.

Il y a quelques dizaines d'années, l'idée avait été émise de réaliser des prothèses, par exemple des prothèses discales comportant une pièce d'interposition souple placée entre deux disques rigides (brevet FR-1.122.634).

Par ailleurs, afin d'éviter ces inconvénients, le Docteur Arthur D. STEFFEE, M.D. a conçu une prothèse dont la structure mécanique autorise à la fois la restitution du soutien et de l'ensemble des mouvements physiologiques du disque (EP-0.392.076). Cela constitue une amélioration remarquable.

Cependant, là encore, il s'agit d'un matériel qui ne peut être mis en place que par un abord chirurgical antérieur et qui n'est conçu que pour un remplacement monobloc et complet d'un disque. A notre connaissance, à l'heure actuelle, ce produit n'a pas été mis à la disposition des chirurgiens.

Le document US-4-772.287 divulgue un disque prothétique et une méthode d'implantation correspondante. Deux disques prothétiques en forme de capsule cylindrique sont implantés l'un à côté de l'autre dans un disque intervertébral endommagé, par formation de deux trous sagittaux respectivement prés des bords latéraux du disque et intervertébral par insertion axiale des disques prothétiques.

La prothése selon l'invention permet de remédier aux inconvénients mentionnés ci-dessus. Sa structure, sa forme et ses dimensions appropriées visent au remplacement d'un demi-disque par pièce prothétique.

Cela permet d'atteindre le triple but mentionné précédemment (restituer la fonction de soutien, restituer la fonction de mouvement, libérer les structures neurologiques) par un seul abord chirurgical postérieur et en un seul temps opératoire, en utilisant éventuellement deux prothèses symétriques pour assurer le remplacement total d'un disque.

La prothèse selon l'invention permet de imiter le risque de traumatisme des structures anatomiques voisines de la prothèse et peut être introduite facilement en position.

A cet effet, l'invention concerne une prothèse discale intervertébrale conforme à la revendication 1.

Dans un mode de réalisation préféré, elle est formée de deux fractions de disque constituant chacune une demi-prothèse.

L'insertion de cette prothèse, possible par abord transcanalaire postérieur, représente un intérêt majeur de l'invention en pratique chirurgicale.

Devant l'indication d'un remplacement discal, le fait de procéder à l'utilisation de deux demi-disques prothétiques ne présente pas d'inconvénient mécanique, dans la mesure où le choix judicieux des dimensions et une pose précise permettront aux deux demi-prothèses contiguës de fonctionner de façon synchrone, réalisant l'équivalent mécanique d'une unité discale monobloc.

Par ailleurs, dans un certain nombre de cas, l'utilisation d'une seule demi-prothèse peut être indiquée, en conservant l'autre moitié du disque naturel, si son état est jugé encore compatible avec une fonction satisfaisante, fonction qui sera, de toute façon, aidée par la demi-prothèse.

Lorsque cela semble faisable, il est ainsi possible d'alléger le geste opératoire.

Ce type de remplacement discal partiel parait particulièrement indiqué :
- en cas de détérioration asymétrique d'un disque, avec pincement discal unilatéral ;
- dans certaines déformations scoliotiques, sur lesquelles l'utilisation judicieuse d'une demi-prothèse, à un ou plusieurs niveaux, devrait permettre une rééquilibration de courbure vertébrale, tout en préservant la fonction.

Elle présente avantageusement les caractéristiques suivantes, éventuellement associées selon toutes leurs combinaisons techniquement possibles :
- sa forme globale parallélépipédique, associée à des dimensions appropriées, autorise une insertion chirurgicale par voie d'abord transcanalaire postérieure du rachis ;
- les plateaux sont en un matériau biocompatible et de forme approximativement rectangulaire ;
- les plateaux comportent chacun des reliefs en épis sur leur face externe, orientés précisément de façon à empêcher le déplacement de la prothèse dans une direction et un sens après son implantation ;
- la surface externe des plateaux entre les épis est rugueuse et présente des aspérités permettant une réhabitation osseuse à partir des surfaces osseuses vertébrales, contre lesquelles elle est appliquée lors de l'implantation chirurgicale ;
- les bords des plateaux sont arrondis dans leur surface et dans leur épaisseur ;
- les plateaux comportent des encoches d'extraction ;
- le coussin est en matériau élastique composite stratifié, ou silicone ou produit de la famille des polyoléfines.

La nécessité éventuelle d'un remplacement discal total, qui n'est pas systématiquement nécessaire suivant les cas pathologiques rencontrés, sera facilement assurée par l'insertion symétrique de deux prothèses hémi-discales à l'étage opéré, l'une sur la partie droite et l'autre sur la partie gauche de l'espace intervertébral considéré.

La description détaillée de l'invention sera faite en référence aux dessins annexés dans lesquels :
- la Figure 1 représente une vue globale en perspective de 3/4 par l'avant d'une prothèse selon l'invention ;
- la Figure 2 représente, en coupe sagittale, la prothèse en place entre deux corps vertébraux.
   (sur chaque dessin : AV = avant ; AR = arrière)

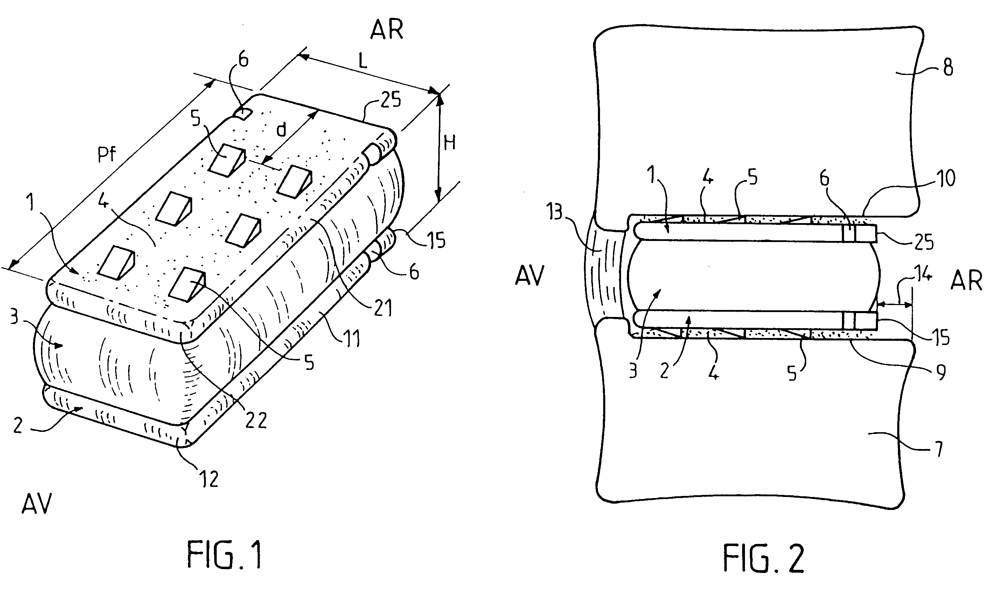

Sur cette Figure 2, n'ont pas été représentées les structures postérieures osseuses, ligamentaires et neurologiques.

Les plateaux métalliques supérieur 1 et inférieur 2 sont constitués d'un métal biocompatible déjà éprouvé dans le domaine de la chirurgie orthopédique.

Leur radio-opacité est un avantage important pour la surveillance post-opératoire du positionnement et de la tenue de la prothèse.

Selon un mode particulier de réalisation, ils peuvent être en titane, qui offre une particularité intéressante dans la mesure où ce matériau est à la fois radio-opaque et compatible avec la réalisation d'examens par résonance magnétique nucléaire, ce qui est très appréciable en cas de nécessité d'explorations endo-canalaires post-opératoires.

Le surfaçage périphérique de ces plateaux métalliques est avantageusement effectué en monobloc en fonderie et procure deux particularités :
- des irrégularités 4 de surface favorisant la réhabitation par le tissu osseux ;
- des structures 5 en relief en forme d'épis, que nous dénommerons "épis anti-recul", dont l'orientation est conçue de façon à éviter la possibilité de migration secondaire de la prothèse de l'avant vers l'arrière, c'est-à-dire de l'espace intervertébral vers le canal vertébral et les structures neurologiques qu'il contient.

Ces deux propriétés de surface des plateaux métalliques contribuent à la stabilité de la prothèse en place, d'une part par accrochage immédiat lors de la pose, d'autre part à terme par le jeu de la réhabitation osseuse.

Les dessins annexés représentent, pour chaque plateau métallique, trois séries de deux épis répartis sur la surface du plateau métallique, cet exemple n'est pas limitatif.

Cependant, la première ligne de positionnement des épis est calculée à une distance d du bord postérieur du plateau métallique permettant l'utilisation d'un portoir spécifique employé pour le positionnement initial de la prothèse entre les corps vertébraux. Une distance d = 7 mm est satisfaisante.

Les plateaux peuvent aussi être constitués, avec les mêmes caractéristiques morphologiques, d'un matériau composite biocompatible de type carbone-graphite, par exemple.

En outre, les plateaux rigides 1, 2 présentent, à l'avant, des bords 11, 21 et des angles 12, 22 (mousses) arrondis, de façon à limiter le risque de traumatisme des structures anatomiques voisines lors de la pose de la prothèse, lequel risque serait beaucoup plus grand en cas de bords et angles vifs.

Les faces arrière 15, 25 sont droites, de façon à faciliter l'introduction de la prothèse à l'aide du portoir venant en appui sur ces faces.

Chaque plateau rigide présente, vers la partie postérieure de ses bords, des encoches dans le métal, dites encoches d'extraction 6.

Ces encoches 6 permettront d'exercer une traction vers l'arrière sur la prothèse, à l'aide d'un instrument approprié, dans l'hypothèse de la nécessité ultérieure d'une réintervention chirurgicale qui imposerait l'ablation de la prothèse.

Le coussin élastique 3 est formé dans un matériau discal présentant des caractéristiques mécaniques se rapprochant au mieux de celles d'un disque naturel, c'est-à-dire qu'il doit répondre aux impératifs des sollicitations physiologiques avec des propriétés d'élasticité et de résistance en fonction des forces de compression d'étirement et de cisaillement, à la fois en rotation et en glissement.

Ces caractéristiques mécaniques sont définies à partir de nombreuses données expérimentales extraites de travaux connus et notoires de biomécanique effectués sur des pièces anatomiques humaines.

Il en ressort que le matériau discal prothétique est réalisé, soit en élastomère de type silicone, soit en matériau composite stratifié, soit en matériau élastique de la famille des polyoléfines.

La prothèse selon l'invention est destinée à pallier les déficiences d'un disque intervertébral dans le maximum de configurations pathologiques possibles.

L'utilisation préférentielle est prévue au niveau du rachis lombaire et la conception de la prothèse autorise, à ce niveau lombaire, sa mise en place préférentielle par voie d'abord rachidienne postérieure transcanalaire, ce type d'abord permettant donc, dans le même temps chirurgical, à la fois le traitement des lésions canalaires postérieures, et le remplacement discal.

Chaque prothèse selon l'invention est prévue pour remplacer la moitié d'un disque lombaire, par la droite ou par la gauche. Elle comporte une fraction de disque recouvrant au plus la moitié de la surface de l'espace discal.

En cas de nécessité, en fonction des lésions discales diagnostiquées, puis constatées pendant l'intervention, l'implantation de deux prothèses, l'une par la droite, l'autre par la gauche, au même étage intervertébral, permet d'assurer un remplacement discal total.

Cette mise en place par abord postérieur nécessite un élargissement canalaire osseux suffisant pour avoir un bon espace d'exposition et une bonne protection des structures neurologiques voisines.

L'insertion entre les corps vertébraux de la prothèse selon l'invention est réalisée, après discectomie appropriée, grâce à la préparation d'un site intercorporéal par creusement des plateaux vertébraux à l'aide d'une instrumentation spécifique.

La Figure 2 représente, en coupe sagittale, la prothèse selon l'invention, en place entre deux corps vertébraux 7, 8, avec ses dispositifs d'accrochage 4, 5, au niveau des plateaux vertébraux osseux 9, 10 avivés, et avec la conservation souhaitable d'une partie antérieure périphérique de l'annulus discal 13, d'une épaisseur antéro-postérieure de l'ordre de 2 à 4 mm.

De plus, le creusement intersomatique et le choix dimensionnel de la prothèse sont calculés, en fonction des caractéristiques anatomiques du cas opéré, de telle sorte à ménager un espace de sécurité 14 de 3 mm minimum, entre l'aplomb du canal vertébral (bord postérieur des corps vertébraux) et la partie postérieure de la prothèse en place.

On s'assure ainsi de l'absence de toute saillie intracanalaire du matériel prothétique.

Dans le but de permettre l'utilisation de la prothèse de l'invention dans différents cas pathologiques, différentes configurations anatomiques, et différents niveaux intervertébraux lombaires, il est prévu un large éventail de tailles de ladite prothèse avec des variations dans ses trois dimensions.

L'expérience pratique opératoire de la mise en place de greffes intersomatiques selon le même mode a conduit à concevoir les variations suivantes, ces exemples n'étant pas limitatifs :
- il est conçu deux tailles de profondeur Pf, l'une de 25 mm, l'autre de 30 mm ;
- dans chacune de ces deux catégories de profondeur, il est prévu des variations de la hauteur H et de la largeur L de la prothèse ;
- pour chacune des deux profondeurs considérées, la hauteur H pourra varier, de millimètre en millimètre, de 9 à 13 ;
- pour chaque profondeur et chaque hauteur citées, la largeur L pourra varier, de millimètre en millimètre, de 9 à 12.

Au niveau du rachis dorsal, l'intérêt de l'utilisation de la prothèse selon l'invention paraît très limité pour deux raisons :
- d'une part, en raison du manque de mobilité naturelle physiologique de ce segment thoracique ;
- d'autre part, en raison des difficultés et des dangers inhérents à la présence de la moëlle épinière qui n'est plus présente au niveau lombaire.

Cependant, il peut être utile et il est possible d'utiliser la prothèse selon l'invention au niveau des segments dorsaux bas D11-D12 et D12-L1, dont la restitution des mobilités peut représenter un intérêt dans certains cas pathologiques.

Les signes de référence insérés après les caractéristiques techniques mentionnées dans les revendications, ont pour seul but de faciliter la compréhension de ces dernières, et n'en limitent aucunement la portée.

## Revendications

1. Prothèse discale intervertébrale destinée à remplacer au moins partiellement un disque intervertébral composé d'un demi-disque gauche et d'un demi-disque droit, chacun desdits demi-disques recouvrant une demi-surface, ladite prothèse comportant un plateau supérieur rigide (1), un plateau inférieur rigide (2), un coussin élastique (3) placé entre le plateau supérieur et le plateau inférieur et comportant une face supérieure et une face inférieure, chacune solidaire du plateau correspondant,
caractérisé en ce qu'elle comporte au moins une fraction de disque recouvrant au plus ladite demi-surface discale de façon à remplacer l'un desdits demi-disques, et en ce que les bords arrière des plateaux sont droits et les bords restants des plateaux sont arrondis de chaque côté et dans leur épaisseur.

2. Prothèse discale intervertébrale selon la revendication 1, caractérisée en ce qu'elle est formée de deux fractions de disque.

3. Prothèse discale intervertébrale selon la revendication 1, caractérisée en ce qu'elle est formée d'une unique fraction de disque.

4. Prothèse discale intervertébrale selon l'une quelconque des revendications 1 à 3, caractérisée par sa forme globale parallélépipédique qui, associée à des dimensions appropriées (Pf, L, H), autorise une insertion chirurgicale par voie d'abord transcanalaire postérieure du rachis.

5. Prothèse discale intervertébrale selon l'une quelconque des revendications 1 à 4, caractérisée en ce que les plateaux (1, 2) ont une forme approximativement rectangulaire.

6. Prothèse discale intervertébrale selon l'une quelconque des revendications 1 à 5, caractérisée en ce que les plateaux (1, 2) comportent chacun des reliefs en épis (5) sur leur face externe, orientés précisément de façon à empêcher le déplacement de la prothèse dans une direction et un sens après son implantation.

7. Prothèse discale intervertébrale selon la revendication 6, caractérisée en ce que la surface externe (4) des plateaux (1, 2) entre les épis (5) est rugueuse et présente des aspérités permettant une réhabitation osseuse à partir des surfaces osseuses vertébrales, contre lesquelles elle est appliquée lors de l'implantation chirurgicale.

8. Prothèse discale intervertébrale selon l'une quelconque des revendications 1 à 7, caractérisée en ce que les plateaux (1, 2) comportent des encoches d'extraction (6).

9. Prothèse discale intervertébrale selon l'une quelconque des revendications 1 à 8, caractérisée en ce que le coussin (3) est en matériau élastique composite stratifié.

10. Prothèse discale intervertébrale selon l'une quelconque des revendications 1 à 8, caractérisée en ce que le coussin (3) est en silicone ou produit de la famille des polyoléfines.

## Claims

1. Intervertebral disk prosthesis intended to replace at least partially an intervertebral disk made of a left half-disk and a right half-disk, each of said half-disk covering a half-surface, said prosthesis comprising a rigid upper plate (1), a rigid lower plate (2), an elastic cushion (3) placed between the upper plate and the lower plate and containing an upper face and a lower face, each attached to the corresponding plate, characterized in that said prosthesis comprises at least one disk fraction covering at most said disk half-surface so as to replace one of said half-disks, and in that the rear edges of the plates are right and the other edges of the plates are rounded at each side, and within their thickness.

2. Intervertebral disk prosthesis according to claim 1, characterized in that it is formed of two disk fractions.

3. Intervertebral disk prosthesis according to claim 1, characterized in that it is formed of a single disk fraction.

4. Intervertebral disk prosthesis according to any of claims 1 to 3, characterized by its global parallelepiped shape which, when its dimensions are appropriate (Pf, L,H) enables surgical insertion by posterior approach through the spinal column.

5. Intervertebral disk prosthesis according to any of claims 1 to 4, characterized in that the plates (1, 2) are approximately rectangular.

6. Intervertebral disk prosthesis according to any of claims 1 to 5, characterized in that each plate (1, 2) has a ledge type of relief (5) on its external face oriented precisely to prevent movement of the prosthesis in a specific direction after its installation.

7. Intervertebral disk prosthesis according to claim 6, characterized in that the external surface (4) of plates (1, 2) between ledges (5) is rough and has sharp edges facilitating rehabitation from vertebral bone surface against which it is applied during the surgical implantation.

8. Intervertebral disk prosthesis according to any of claims 1 to 7, characterized in that the plates (1, 2) comprise extraction notches (6).

9. Intervertebral disk prosthesis according to any of claims 1 to 8, characterized in that the cushion (3) is made of a laminated composite elastic material.

10. Interbertebral disk prosthesis according to any of claims 1 to 8, characterized in that the cushion (3) is made of silicone or a product in the polyolefines family.

## Patentansprüche

1. Bandscheibenprothese zum wenigstens teilweisen Ersetzen einer aus einer linken Scheibenhälfte und einer rechten Scheibenhälfte bestehenden Bandscheibe, wobei jede der Scheibenhälften eine Flächenhälfte abdeckt, wobei die Prothese eine starre obere Platte (1), eine starre untere Platte (2) und ein zwischen der oberen Platte und der unteren Platte angeordnetes elastisches Kissen (3) mit einer Oberseite und einer Unterseite aufweist, die jeweils fest mit der entsprechenden Platte verbunden sind,
dadurch gekennzeichnet, daß die Prothese wenigstens einen Scheibenteil aufweist, der höchstens die Flächenhälfte der Scheibe derart abdeckt, daß sie eine der Scheibenhälften ersetzt, und daß die hinteren Ränder der Platten gerade und die übrigen Ränder der Platten auf jeder Seite und in ihrer Dicke gerundet sind.

2. Bandscheibenprothese nach Anspruch 1, dadurch gekennzeichnet, daß sie aus zwei Scheibenteilen gebildet ist.

3. Bandscheibenprothese nach Anspruch 1, dadurch gekennzeichnet, daß sie aus einem einzigen Scheibenteil gebildet ist.

4. Bandscheibenprothese nach einem der Ansprüche 1 bis 3, gekennzeichnet durch ihre generell parallelepipedische Form, die, in Verbindung mit den geeigneten Abmessungen (Pf, L, H), ein operatives Einsetzen über den hinteren transkanalären Zugang der Wirbelsäule ermöglicht.

5. Bandscheibenprothese nach einem der Ansprüche 1 bis 4, dadurch gekennzeichnet, daß die Platten (1, 2) ungefähr rechteckige Form haben.

6. Bandscheibenprothese nach einem der Ansprüche 1 bis 5, dadurch gekennzeichnet, daß die Platten (1,2) auf der Außenseite ein Rippenrelief (5) aufweisen, dessen Rippen genau so ausgerichtet sind, daß sie nach dem Einsetzen ein Verschieben der Prothese in einer Richtung und einer Drehrichtung verhindern.

7. Bandscheibenprothese nach Anspruch 6, dadurch gekennzeichnet, daß die Außenfläche (4) der Platten (1, 2) zwischen den Rippen (5) rauh ist und asphärische Ausformungen aufweist, die eine von den Wirbelknochenflächen, an denen die Prothese beim operativen Einsetzen angelegt wird, ausgehende Knochenrehabitation ermöglichen.

8. Bandscheibenprothese nach einem der vorhergehenden Ansprüche 1 bis 7, dadurch gekennzeichnet, daß die Platten (1, 2) Extraktionsausnehmungen (8) aufweisen.

9. Bandscheibenprothese nach einem der Ansprüche 1 bis 8, dadurch gekennzeichnet, daß das Kissen (3) aus elastischem Schichtverbundmaterial besteht.

10. Bandscheibenprothese nach einem der Ansprüche 1 bis 8, dadurch gekennzeichnet, daß das Kissen (3) aus Silikon oder einem Produkt aus der Familie der Polyolefine besteht.
